## Europäisches Patentamt
## European Patent Office
## Office européen des brevets

(11) Veröffentlichungsnummer: **0 008 808**
**A1**

(12) # EUROPÄISCHE PATENTANMELDUNG

(21) Anmeldenummer: **79103412.7**

(22) Anmeldetag: **12.09.79**

(51) Int. Cl.³: **G 01 N 33/12**
**C 12 Q 1/24**

(30) Priorität: **13.09.78 DE 2839702**

(43) Veröffentlichungstag der Anmeldung:
**19.03.80 Patentblatt 80/6**

(84) Benannte Vertragsstaaten:
**AT BE CH FR GB IT LU NL SE**

(71) Anmelder: **Reuter, Adolf, Ing. (grad.)**
**Am Grassenberg 6**
**D-3550 Marburg/Lahn(DE)**

(72) Erfinder: **Reuter, Adolf, Ing. (grad.)**
**Am Grassenberg 6**
**D-3550 Marburg/Lahn(DE)**

(74) Vertreter: **Olbricht, Karl Heinrich, Dipl.-Phys.**
**Am Weinberg 15**
**D-3551 Niederweimar(DE)**

(54) **Verfahren und Vorrichtung zur Keimzahlbestimmung an Oberflächen.**

(57) Die Erfindung dient zur reproduzierbaren Keimzahlbestimmung an Oberflächen durch Abspülen einer wählbaren Prüffläche (P) eines zu untersuchenden Stückes insbesondere mit Wasser (W). Die Prüffläche (P) wird durch dichtendes Andrücken des Öffnungsrandes (20) eines Behälters (12) an das zu untersuchende Stück definiert. Dann wird der Keimgehalt des im unteren Behälter-Teil (16) aufgefangenen Spülwassers (W) gemessen, das zuvor zweckmäßig filtriert und gekühlt wird, um eine zusätzliche Trübung durch Fett- und Faserteilchen zu verhindern. Man füllt durch eine Öffnung (18) eine vorgegebene Wassermenge ein und bewegt sie in dem vom Behälter (12) und der Prüffläche (P) begrenzten Volumen eine vorgegebene Zeit lang periodisch mittels eines Förderelements (22), das mit Abstand zu der Öffnung (18, 64) im Behälter (12) wenigstens teilweise eintauchend angeordnet ist. Letzterer kann Bestandteil eines abnehm- und sterilisierbaren Gerätekopfes sein, an dessen Öffnung (28) eine mit einer Außenöffnung (64) versehene Abdeckblende (62) austauschbar angebracht ist. Ein elektronisch gesteuerter Antrieb (32) bewegt das Förderelement (22). Dieses kann wenigstens einen Flügel (24) haben, der z.B. über eine den Behälter (12) abgedichtet durchsetzende oder an ihn heranreichende Drehachse (30), gegebenenfalls mit einer Magnet(rutsch)kupplung (46) rotierbar ist.

./...

Fig. 1

Dipl.-Phys. Karl H. Olbricht
Patentanwalt
Am Weinberg 15
D-3551 Niederweimar
Telefon (06421) 78627

0008808

PH 288          Ot/Hg

Ing. Adolf Reuter, am Grassenberg 6, 3550 Marburg/Lahn

---

## Verfahren und Vorrichtung zur Keimzahlbestimmung an Oberflächen

---

### B e s c h r e i b u n g

Die vorliegende Erfindung betrifft ein Verfahren und eine
Vorrichtung zur Keimzahlbestimmung an Oberflächen, z.B. an
zu verarbeitendem Fleisch, durch Abspülung einer wählbaren
Prüffläche eines zu untersuchenden Stückes mit einer Flüssigkeit, insbesondere Wasser, und durch anschließende Messung
des Keimgehaltes der aufgefangenen Spülflüssigkeit beispielsweise mittels Trübe-Bestimmung, mikroskopischer Auszählung
oder Impfung auf Nährböden.

Für zahlreiche Zwecke ist es geboten bzw. erwünscht, eine
einmalige oder wiederholte Untersuchung von Oberflächen
durchzuführen, um die daran haftenden Keime zu ermitteln. So
bediente man sich zur Prüfung der Qualität angelieferten
Fleisches vor der Weiterverarbeitung verschiedener mehr oder
weniger aufwendiger Methoden. Ein Beispiel hierfür ist der
sog. Nitrat-Reduktase-Test, bei dem aus der Reduktionszeit
von Nitrat auf die Anzahl reduktionsfördernder Mikroorganismen
geschlossen wird. Derartige Verfahren erfordern geschultes Personal sowie zum Teil recht aufwendige Geräte und entsprechende Reagenzien, was die Qualitätsbestimmung stark verteuert. Für Laborzwecke sind diese Methoden anwendbar, unter Praxisbedingungen jedoch wenig geeignet.

Besser eignet sich z.B. zur Untersuchung von Fleisch die Abschwemm-Methode, die schnell und einfach durchzuführen ist. Dazu müssen gleich große Flächen der zu prüfenden Stücke jeweils mit gleicher Intensität und gleicher Menge eines Spülmittels, vorzugsweise Wasser, abgeschwemmt werden, um miteinander vergleichbare Werte für die Keimzahlbestimmung zu erhalten. Diese kann durch ATP-Messung erfolgen, indem in einer solchen Fleischabschwemmung der Gehalt an Adenosintriphosphat, der in Zellen verschiedener Bakterienarten relativ konstant ist, insbesondere auf optischem Wege ermittelt wird.

Unter Beachtung dieser herkömmlich nicht oder nur unzureichend erfüllten Voraussetzungen ist es Aufgabe der Erfindung, die Nachteile des Standes der Technik mit einfachen, wirtschaftlichen Mitteln zu überwinden und die Keimzahlbestimmung an zu prüfenden Abschwemm-Oberflächen rasch, vor allem aber zuverlässig und möglichst genau zu ermöglichen, und zwar gerade auch durch ungeschultes Personal sowie unter den verschiedensten Arbeits- und Umgebungs- bzw. Hygiene-Bedingungen.

Bei einem Verfahren der eingangs genannten Art ist erfindungsgemäß vorgesehen, daß in einen Behälter durch eine Öffnung eine vorgegebene Wassermenge eingefüllt und die Prüffläche durch dichtendes Andrücken des Öffnungsrandes an das zu untersuchende Stück definiert wird, während die Wassermenge in dem vom Behälter und der Prüffläche begrenzten Volumen eine vorgegebene Zeit lang periodisch bewegt wird, worauf die Sammlung des Spülwassers in dem Behälter erfolgt. Die Wassermenge kann während des Spülvorganges im Kreislauf bewegt,
z.B. motorisch umgewälzt oder umgepumpt, und anschließend
in einem unteren Teil des Behälters gesammelt werden.
Vorzugsweise wird das aufgefangene Spülwasser vor der
Keimgehalts-Messung gekühlt und dann filtriert, z.B.
mit einem keimdurchlässigen Faltenfilter, um von der
Prüffläche mit abgeschwemmte Partikel zuverlässig aus der
zu messenden Spülflüssigkeit zu entfernen.

Die erfindungsgemäße Vorrichtung weist einen Behälter mit
Begrenzungskanten für eine Öffnung und im Abstand zu
letzterer wenigstens ein Förderelement auf, mit dem die
Spülflüssigkeit während einer vorgebbaren Zeit periodisch
an die Prüffläche bewegbar ist. Der Behälter kann Bestandteil
eines abnehm- und/oder sterilisierbaren Gerätekopfes sein.
In diesem bewegt sich das Förderelement, beispielsweise ein
mit gleichbleibender Drehzahl während einer bestimmten Zeit
rotierender Flügel. Dadurch wird die in einen unteren Teil
des Behälters eingemessene Spülwassermenge an die Öffnung
des Gerätekopfes geschleudert, die bei der Anwendung dicht
an der zu prüfenden Oberfläche anliegt. Das Wasser läuft
unter Abspülung der Prüffläche wieder in den unteren Teil
des Gerätekopfes, wo es vom Förderelement erfaßt und erneut
an die Prüffläche gefördert wird. Der so gebildete Wasserkreislauf reichert sich mit den auf der Oberfläche befindlichen Keimen rasch an. Vorteilhaft ist hier vor allem, daß
Keime auch aus der Tiefe von Oberflächen-Unebenheiten wie
Falten, Rillen, Poren usw. herausgespült werden, was ein
wichtiger Fortschritt gegenüber herkömmlichen Abdruck-
Methoden ist, bei denen ein Substrat auf die Prüffläche
gedrückt, abgehoben und untersucht wird; die Zahl der dabei
mitgenommenen und erfaßten Bakterien war jedoch von zufälligen
Gegebenheiten und Einflüssen stark abhängig, so daß die
Keimzahlbestimmung bisher entsprechend unsicher war.

Nach der vorgewählten Laufzeit schaltet eine elektronische Steuerung den Antrieb des erfindungsgemäßen Gerätes automatisch ab. Dadurch ist sichergestellt, daß stets gleiche Abschwemmbedingungen an einer beliebig wählbaren Prüffläche herrschen, deren Größe durch den Öffnungsrand des Gerätekopfes oder einer daran befestigbaren Abdeckblende eindeutig definiert ist. Dieser Rand ist vorzugsweise scharfkantig und/oder mit einer Dichtungsauflage versehen, um jeglichen Verlust an Spülflüssigkeit zu vermeiden. Die anschließende Keimzahl-Bestimmung, beispielsweise in einem geeigneten Trübe-Meßgerät, liefert daher gut reproduzierbare Werte.

Versuche haben gezeigt, daß die Erfindung für die hygienische Beurteilung von Oberflächenzuständen, z.B. an angeliefertem Rohfleisch, vorzüglich geeignet ist. Im Regelfalle genügt eine Laufzeit von 30 s zur intensiven Abschwemmung der zu untersuchenden Oberfläche. Das Volumen der Spülflüssigkeit richtet sich nach der Größe der Prüffläche; es kann beispielsweise 20 ml für eine Prüffläche der Größenordnung 40 cm$^2$ betragen. Da der Gerätekopf, welcher zugleich den Behälter für die Prüfflüssigkeit bildet, samt Förderelement vorzugsweise abnehmbar ist, besteht eine vorteilhafte Ausgestaltung der Erfindung darin, mehrere Geräteköpfe unterschiedlichen Fassungsvermögens und unterschiedlicher Öffnungsgröße vorzusehen. Man kann aber auch verschiedene, z.B. aufsteckbare Abdeckblenden an der Öffnung eines einzigen Gerätekopfes anbringen, um kleinere oder anders begrenzte Außenöffnungen zu erhalten. Verschiedene Vorsätze definieren dann unterschiedlich große und/oder unterschiedlich gestaltete Prüfflächen, gegebenenfalls auch Pegelkanten. Durch Austausch des Kopfes oder der Abdeckblende ist daher eine bequeme Anpassung an geänderte Prüfbedingungen möglich. Wenn Kopf und Förderelement aus Metall bestehen, ist nicht nur die Reinigung, sondern auch die Sterilisierung im Autoklaven erleichtert, die insbesondere bei Temperaturen von etwa 180 $^o$C vor sich gehen kann.

Zahlreiche weitere Merkmale und Vorteile der Erfindung
ergeben sich aus den Ansprüchen und der nachfolgenden
Beschreibung von Ausführungsbeispielen anhand der Zeichnung.
Darin zeigen:

Fig.  1  eine schematisierte Seitenansicht, teilweise im
         Schnitt, einer erfindungsgemäßen Vorrichtung,

Fig.  2  eine Stirnansicht des Gerätekopfes von Fig. 1,

Fig.  3  eine Schräganr icht einer erfindungsgemäßen
         Vorrichtung zur Veranschaulichung der Anwendung,

Fig.  4  eine Teil-Seitenansicht, teilweise im Schnitt,
         einer abgewandelten Ausführungsform bei Andruck
         an eine Prüffläche und

Fig.  5  eine Teil-Seitenansicht, teilweise im Schnitt,
         noch einer anderen Ausführungsform einer
         Vorrichtung gemäß der Erfindung.

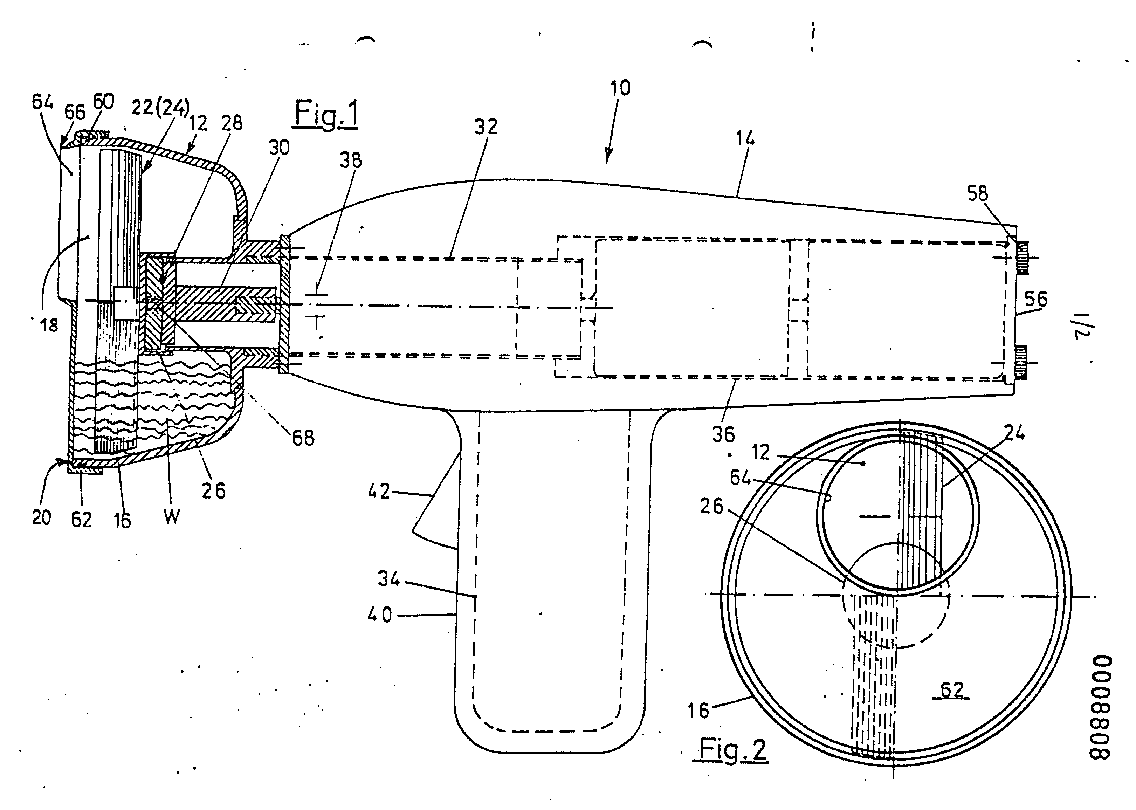

Im Ausführungsbeispiel der Fig. 1 und 2 besteht das insgesamt
mit 10 bezeichnete Gerät aus einem als Behälter ausgebildeten
Gerätekopf 12 und einem Hauptteil 14 mit Handgriff 40. Der
abnehmbare Gerätekopf 12 stellt einen Behälter dar, dessen
unterer Teil 16 zur Aufnahme einer Spülwassermenge W dient.
Um diese in einfacher Weise bemessen zu können, kann die
von einer Randkante 20 begrenzte Öffnung 18 des Behälters 12
an der Unterkante M eine Marke tragen oder geradlinig ausgebildet sein, so daß ein Meßpegel vorgegeben ist. Man kann
aber auch ein bestimmtes Volumen z.B. mit einer Injektionsspritze exakt bemessen und einfüllen.

Gegenüber der Öffnung 18 ist im Behälter 12 ein Förderelement
22 angeordnet, das im Beispiel der Fig. 1 und 2 als Flügel 24
mit einer Nabe 26 gestaltet ist, die mittels einer Magnetkupplung 28 in einfach lösbarer Weise mit einer Drehachse 30
eines Antriebes 32 verbindbar bzw. verbunden ist. Der beispielsweise als Motor mit Untersetzungsgetriebe ausgebildete
Antrieb 32 und der elektrische Speiseteil 36 können im Oberteil des Geräte-Hauptteils 14 untergebracht sein, der durch
einen Deckel 56 mit Rändelschrauben 58 oder Muttern abgeschlossen ist. Der Griff 40 nimmt bevorzugt einen Steuerteil
34 auf. Außerdem können (nicht gezeichnete) Anzeigelämpchen
für die Betriebskontrolle und, wenn der Speiseteil 36
Batterien bzw. Akkumulatoren aufweist, für deren Ladezustand
vorhanden sein. Wenn das Gerät 10 zur Speisung oder Nachladung
aus dem Elektrizitätsnetz eingerichtet ist, kann - vorzugsweise
am unteren Ende des Handgriffes 40 - ein Anschlußkabel 54
angebracht sein, das aus Sicherheitsgründen nur Niederspannung
führt und zur Verbindung mit einem (nicht dargestellten)
Netzteil dient. Ein Schalter 42 ist bevorzugt nach Art eines
Pistolen-Abzuges ausgebildet; er kann ein Taster oder auch
ein während des Betriebes eingerasteter Druckschalter sein.

Besonders einfach und kostengünstig ist es, wenn das Förderelement 22 zweiflügelig und der Flügel 24 in der Weise
teilzylindrisch ist, daß jeweils ein Stück einer zylindrischen
Mantelfläche mit der Hohlseite zur Öffnung 18 hin an der
Nabe 26 angebracht ist. Diese kann zugleich eine Überfangkappe bilden, die das Austreten von Spülwasser zu den
Antriebselementen unterbindet, an denen wahlweise oder
zusätzlich eine Wellendichtung 38 vorgesehen sein kann,
etwa eine fettgetränkte Sintermetallbuchse o. dgl.

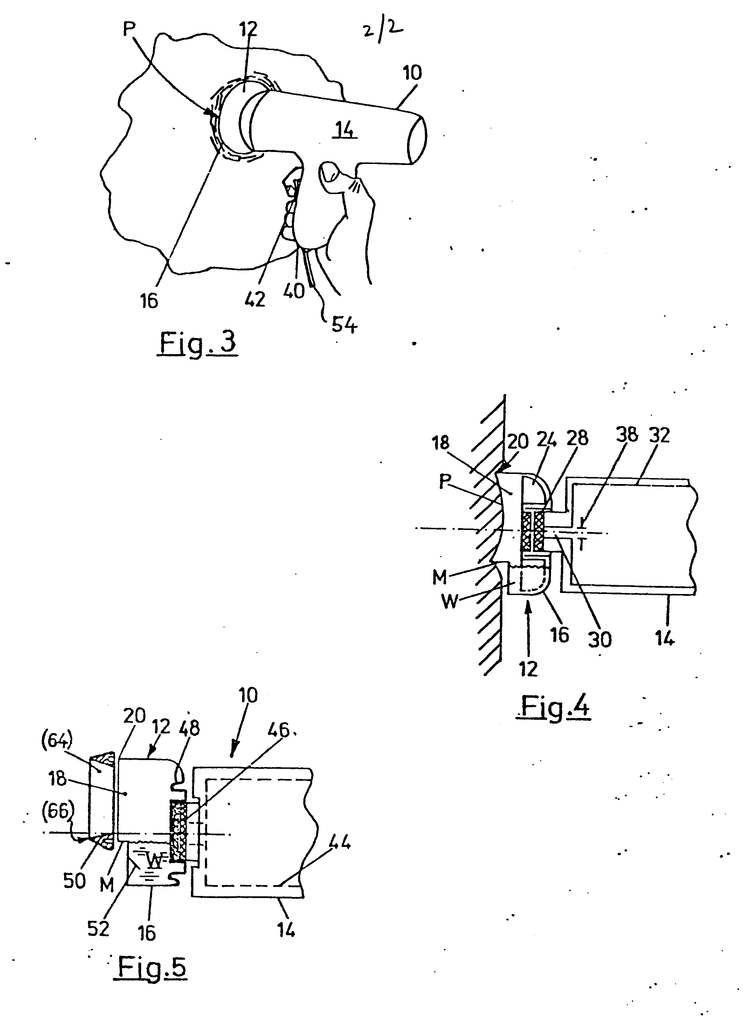

Um vergleichbare Prüfergebnisse zu sichern, sieht die
Erfindung eine konstante Größe der Öffnung 18 vor, deren
Randkante 20, gegebenenfalls mit aufgesteckter Dichtung 50
(Fig. 5), die Prüffläche P stets gleich begrenzt. Eine solche
konstante Begrenzung ist auch mit Vorsatzplatten erzielbar,
die in Form von Abdeckblenden 62 (Fig. 1 und 2) auf den mit
einer Ringdichtung 60 versehenen Rand des Behälters 12
aufgesteckt oder nach Art eines Bajonettverschlusses aufgeschraubt werden. Wiederum definieren dann Randkanten 66,
gegebenenfalls mit aufgesteckter Dichtung 50, die Außenöffnung
64 und mithin die Prüffläche P. Auch die Unterkante der Außenöffnung 64 kann zugleich eine Marke für den Füllpegel der
Spülflüssigkeit W bilden. Durch Abnehmen oder Auswechseln der

Abdeckblenden 62 läßt sich das Gerät 10 rasch den unterschiedlichsten Bedürfnissen anpassen.

Der Antrieb 32 weist vorzugsweise einen Generator auf. Wichtig ist eine Zentrierung 66 zwischen Drehachse 30, Magnetkupplung 28 und/oder Nabe 26, dank deren das Förderelement 22 gleichmäßig und reibungsarm umläuft. Es wird infolge der Steuerung während einer einstellbaren Zeit periodisch angetrieben, so daß die Bewegung der Spülwassermenge W im Volumen des Behälters 12 mit einer vorgebbaren Gesamtzahl von Perioden erfolgt. Mit Rücksicht auf die mechanischen Eigenschaften des Spülwassers W wird dabei eine verhältnismäßig niedrige Bewegungsfrequenz gewählt, so daß die vom Förderelement 22 bewegte Wassermenge jeweils Zeit hat, von der Prüffläche P in den Behälter-Unterteil 16 fortlaufend zurückzuströmen. Außerdem ist der Flügel 24 samt seiner Nabe 26 in solchem Abstand zu der Öffnung 18 bzw. 64 angeordnet, daß auch eine unter dem Andruck des Gerätes 10 konvex gewölbte Prüffläche P die Bewegung des Flügels 24 nicht zu hemmen bzw. zu behindern vermag (vergl. Fig. 4).

Die einfache Handhabung des Gerätes 10 ist aus Fig. 3 ersichtlich. Man drückt es mit dem Handgriff 40 auf das zu prüfende Stück und betätigt den Schalter 42. Wenn nach der vorgegebenen Laufzeit von z.B. 30 s der Antrieb 32 automatisch abgeschaltet und das Förderelement 22 zum Stillstand gekommen ist, wartet man noch wenige Sekunden, bis im Unterteil des Behälters 12 die Spülwassermenge W gesammelt ist. Dann kann das Gerät 10 von der Prüffläche P abgehoben und die Spülwassermenge W zur eigentlichen Keimzahlbestimmung entnommen werden, am einfachsten durch Umkippen des Gerätes 10, so daß die Flüssigkeit durch die Öffnung 18 bzw. 64 in einen Auffangbehälter ausläuft.

Gemäß dem abgewandelten Ausführungsbeispiel der Fig. 4 ist der Behälter 12 als verhältnismäßig flache Dose ausgebildet, wobei der von der Meßkante M begrenzte Unterteil 16 in Axialrichtung weitgehend oder ganz von dem Flügel 24 ausgefüllt wird. Dessen Vorderseite hat wiederum von der Prüffläche P in der durch die Randkante 20 begrenzten Öffnung 18 einen ausreichenden Sicherheitsabstand. ·

Noch eine weitere Ausführungsform ist in Fig. 5 dargestellt. Hierbei beherbergt der Hauptteil 14 des Gerätes 10 einen Schwingantrieb 44, der über eine Magnetkupplung 46 mit dem abnehmbaren Gerätekopf 12 verbindbar ist. Dieser hat eine Membran 48, die formschlüssig an die Magnetkupplung 46 anschließt. Durch Betätigung des Schwingantriebes 44 wird die im Behälter-Unterteil 16 gesammelte Spülwassermenge W zunächst axial bewegt, wobei infolge des begrenzten Behältervolumens rasch radiale Bewegungskomponenten hinzutreten. Die von der Randkante 20 begrenzte Öffnung 18 und mithin die Prüffläche P des zu untersuchenden Objektes wird daher während der Laufzeit des Schwingantriebes 44 ständig mit Wasser W beschickt, was durch Prallflächen 52 noch unterstützt werden kann. - Bei dieser konstruktiv besonders einfachen Ausführungsform kann der Gerätekopf unter Umständen einstückig mit der Membran 48 hergestellt werden, beispielsweise aus steifem Gummi oder hochelastischem Weichkunststoff. Statt eines mechanischen Schwingantriebes kann auch ein Ultraschall- oder Düsenantrieb vorhanden sein.

Für die Antriebssteuerung nach der Erfindung kann ein Impulsgenerator mit einer Zähleinrichtung verwendet werden, die nach einer voreinstellbaren Gesamtzahl von Impulsen für die Abschaltung des Antriebes 32 bzw. 44 sorgt. Wenn beispielsweise 32 Impulse je Umdrehung des Förderelementes 22 bei 20 U/s während einer Laufzeit von 30 s erzeugt werden, sind das insgesamt 19.200 Impulse. Verändert sich nun die Gleichmäßigkeit des Umlaufes, aus welchem Grunde auch immer, dann hat dies keinerlei Einfluß auf die gerade durchgeführte Messung, weil die elektronische Antriebssteuerung 34 in diesem Falle die Laufzeit automatisch entsprechend verändert, nämlich verkürzt oder verlängert bis zum Ablauf der eingestellten Impulszahl. Analog kann die Antriebssteuerung 34 auf einen Schwingantrieb 44 des Ausführungsbeispiels von Fig. 5 wirken, für den zweckmäßig ebenfalls eine Untersetzung vorgesehen ist, welche die zuverlässige Abspülung der Prüffläche P gewährleistet.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

0008808

Dipl.-Phys. Karl H. Olbricht
Patentanwalt
Am Weinberg 15
D-3551 Niederweimar
Telefon (06421) 78627

PH 288    Ot/Hg

Ing. Adolf Reuter, am Grassenberg 6, 3550 Marburg/Lahn

Verfahren und Vorrichtung zur
Keimzahlbestimmung an Oberflächen

P a t e n t a n s p r ü c h e

1. Verfahren zur Keimzahlbestimmung an Oberflächen, z.B. an
zu verarbeitendem Fleisch, durch Abspülung einer wählbaren
Prüffläche eines zu untersuchenden Stückes mit einer
Flüssigkeit, insbesondere Wasser, und durch anschließende
Messung des Keimgehaltes der aufgefangenen Spülflüssigkeit
beispielsweise mittels Trübe-Bestimmung, mikroskopischer
Auszählung oder Impfung auf Nährböden, dadurch  g e k e n n -
z e i c h n e t , daß in einen Behälter durch eine Öffnung
eine vorgegebene Wassermenge eingefüllt und die Prüffläche
durch dichtendes Andrücken des Öffnungsrandes an das zu
untersuchende Stück definiert wird, während die Wassermenge
in dem vom Behälter und der Prüffläche begrenzten Volumen eine
vorgegebene Zeit lang periodisch bewegt wird, worauf die
Sammlung des Spülwassers in dem Behälter erfolgt.

2. Verfahren nach Anspruch 1, dadurch g e k e n n z e i c h ‐ n e t , daß die Wassermenge während des Spülvorganges im Kreislauf bewegt, z.B. motorisch umgewälzt oder umgepumpt, und anschließend in einem unteren Teil des Behälters gesammelt wird.

3. Verfahren nach Anspruch 1 oder 2, dadurch g e k e n n ‐ z e i c h n e t , daß das aufgefangene Spülwasser vor der Keimgehalts-Messung filtriert wird, z.B. mit einem keimdurchlässigen Faltenfilter.

4. Verfahren nach Anspruch 3, dadurch g e k e n n z e i c h ‐ n e t , daß das Spülwasser gekühlt oder gekühltes Spülwasser verwendet wird.

5. Vorrichtung zur Keimzahlbestimmung an Oberflächen, z.B. an zu verarbeitendem Fleisch, durch Abspülung einer wähl‐ baren Prüffläche eines zu untersuchenden Stückes mit einer Flüssigkeit, insbesondere Wasser, und durch anschließende Messung des Keimgehaltes der aufgefangenen Spülflüssigkeit beispielsweise mittels Trübe-Bestimmung oder mikroskopischer Auszählung oder Impfung auf Nährböden, dadurch g e k e n n ‐ z e i c h n e t , daß ein Behälter (12) mit Begrenzungs‐ kanten (20 bzw. 66) für eine Öffnung (18 bzw.64) vorhanden und mit Abstand zu letzterer im Behälter (12) wenigstens ein Förderelement (22) angeordnet ist, mit dem die Spül‐ flüssigkeit (W) während einer vorgebbaren Zeit periodisch an die Prüffläche (P) bewegbar ist.

6. Vorrichtung nach Anspruch 5, dadurch g e k e n n z e i c h ‐ n e t , daß der Behälter (12) Bestandteil eines abnehm‐ und/oder sterilisierbaren Gerätekopfes ist.

7. Vorrichtung nach Anspruch 5 oder 6, dadurch g e k e n n ‐ z e i c h n e t , daß ein unterer Teil (16) des Behälters (12) zum Auffangen der Spülwassermenge dient, in die das Förderelement (22) wenigstens teilweise eintaucht.

8. Vorrichtung nach einem der Ansprüche 5 bis 7, dadurch g e k e n n z e i c h n e t , daß die Öffnung (18) des Behälters (12) von einer nach außen vorstehenden, vorzugsweise scharfen Randkante (20) begrenzt ist.

9. Vorrichtung wenigstens nach Anspruch 8, dadurch g e - k e n n z e i c h n e t , daß die Randkante (20) am unteren Teil (16) des Behälters (12) geradlinig verläuft.

10. Vorrichtung nach wenigstens einem der Ansprüche 5 bis 9, dadurch g e k e n n z e i c h n e t , daß an der Öffnung (28) des Behälters (12) eine Abdeckblende (62) austauschbar angebracht ist, die ihrerseits eine Außenöffnung (64) hat.

11. Vorrichtung nach Anspruch 10, dadurch g e k e n n - z e i c h n e t , daß die Außenöffnung (64) der Abdeckblende (62) von einer nach außen vorstehenden, vorzugsweise scharfen Randkante (66) begrenzt ist, insbesondere kreisrund, oval und/oder unten geradlinig.

12. Vorrichtung nach Anspruch 10 oder 11, dadurch g e k e n n z e i c h n e t , daß die Begrenzung der Öffnung (18 bzw. 64) als konischer oder zylindrischer Dichtungsrand ausgebildet ist, gegebenenfalls mit einer Ringnut (60) zur Aufnahme von Dichtungsmaterial, und daß die Abdeckblende (62) einen formschlüssigen Konus- oder Zylinderrand aufweist.

13. Vorrichtung nach wenigstens einem der Ansprüche 5 bis 12, dadurch g e k e n n z e i c h n e t , daß an dem Außenrand der Öffnung (18) oder der Außenöffnung (64) eine insbesondere elastisch-nachgiebige Dichtung (50) anbringbar ist.

0008808

14. Vorrichtung nach wenigstens einem der Ansprüche 10 bis 13, dadurch g e k e n n z e i c h n e t , daß die Abdeckblende (62) am Behälterrand in ihrer Lage fixierbar ist, z.B. mittels Stift und Kerbe, Kugelrast o. dgl.

15. Vorrichtung nach wenigstens einem der Ansprüche 8 bis 14, dadurch g e k e n n z e i c h n e t , daß zumindest die Randkante (20, 66) des Behälters (12) und/oder der Abdeckblende (62) aus elastisch-nachgiebigem Material besteht.

16. Vorrichtung nach wenigstens einem der Ansprüche 5 bis 15, dadurch g e k e n n z e i c h n e t , daß das Förderelement (22) mit einer einstellbaren Anzahl von Perioden bewegbar ist.

17. Vorrichtung nach wenigstens einem der Ansprüche 5 bis 16, dadurch g e k e n n z e i c h n e t , daß für die Bewegung des Förderelements (22) ein elektronisch gesteuerter Antrieb (32) vorhanden ist.

18. Vorrichtung nach Anspruch 17, dadurch g e k e n n z e i c h n e t , daß der Antrieb ein insbesondere batteriegespeister Motor ist, vorzugsweise mit Untersetzungsgetriebe.

19. Vorrichtung nach Anspruch 17 oder 18, dadurch g e k e n n z e i c h n e t , daß die Antriebssteuerung (34) Einrichtungen aufweist, mit denen während eines wählbaren Zeitraums Impulse erzeug- und zählbar sind.

20. Vorrichtung nach wenigstens einem der Ansprüche 5 bis 19, dadurch g e k e n n z e i c h n e t , daß das Förderelement (22) wenigstens einen Flügel (24) aufweist, der über eine den Behälter (12) abgedichtet durchsetzende oder an ihn unmittelbar heranreichende Drehachse (30) rotierend antreibbar ist.

21. Vorrichtung nach Anspruch 20, dadurch g e k e n n - z e i c h n e t , daß die Nabe (26) des Flügels (24) und die Drehachse (30) durch eine Rutschkupplung lösbar antriebsverbunden sind, vorzugsweise eine Magnetkupplung (28) mit Zentrierung (68).

22. Vorrichtung nach Anspruch 20 oder 21, dadurch g e k e n n - z e i c h n e t , daß der insbesondere an die benachbarte Behälterwand formangepaßte Flügel (24) einsinnig zur Öffnung (18) hin gehöhlte Arme, Schaufeln o. dgl. aufweist.

23. Vorrichtung nach wenigstens einem der Ansprüche 5 bis 22, dadurch g e k e n n z e i c h n e t , daß das Förder-element (22) eine Membran (48) ist, die über eine lösbare Magnetkupplung (46) von einem Schwingantrieb (44) aus bewegbar ist.

24. Vorrichtung nach Anspruch 23, dadurch g e k e n n - z e i c h n e t , daß die Membran (48) mit der Behälterwandung verbunden oder mit ihr einstückig ist.

25. Vorrichtung nach wenigstens einem der Ansprüche 5 bis 24, dadurch g e k e n n z e i c h n e t , daß ein Handgriff (40) mit Druckschalter (42) für die Antriebssteuerung (34) vorhanden ist.

26. Vorrichtung nach Anspruch 25, dadurch g e k e n n - z e i c h n e t , daß die Antriebssteuerung (34) in dem Handgriff (40) untergebracht ist.

27. Vorrichtung nach wenigstens einem der Ansprüche 6 bis 26, dadurch g e k e n n z e i c h n e t , daß zwischen Gerätekopf bz. Behälter (12) und Hauptgerät (14) wenigstens eine Dichtung vorgesehen ist, insbesondere als oder mit Überfangkappe (26), als Wellendichtung (28) o. dgl.

28. Vorrichtung nach wenigstens einem der Ansprüche 16 bis 27, dadurch g e k e n n z e i c h n e t , daß vorzugsweise im oberen Teil des Hauptgerätes (14) ein Speiseteil (34) mit Batterien und/oder einem Netzanschlußgerät für den Antrieb (32, 44) angeordnet ist.

29. Vorrichtung nach wenigstens einem der Ansprüche 5 bis 28, dadurch g e k e n n z e i c h n e t , daß der Gerätekopf (12) am Hauptteil (14) des Gerätes (10) axialbeweglich angebracht und durch dessen Andrücken an die Prüffläche (P) ein Antriebsschalter betätigbar ist.

0008808

Legende PH 288

M    Meßkante

P     Prüffläche

W    Spülwasser(menge)

10   Gerät

12   Gerätekopf/Behälter

14   Hauptteil

16   Behälter-Unterteil

18   Öffnung

20   Randkante

22   Förderelement

24   Flügel

26   Nabe = Überfangkappe

28   Magnetkupplung

30   Drehachse

32   Antrieb(smotor)

34   Steuerteil

36   Speiseteil

38   Wellendichtung

40   Handgriff

42   Druckschalter

44   Schwingantrieb

46   Magnetkupplung

48   Membran

50   Dichtung

52   Leitflächen

54   Anschlußkabel

56   Deckel

58   Rändelschrauben

60   Ringdichtung

62   Abdeckblende

64   Außenöffnung

66   Randkante

68   Zentrierung

Fig.1

Fig.2

1/2

8008808

0008808

2/2

Fig.3

Fig.4

Fig.5

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A - 2 618 334 (M. KORBIN)<br>* Seite 11, Zeile 1 - Seite 12, Zeile 3; Figur 1 *<br>-- | 1 |
| A | FR - A - 2 366 554 (BATTELLE INSTITUT E.V.)<br>* Seite 7, Zeilen 1-9 *<br>-- | 1 |
| A | US - A - 3 362 141 (G.W. ROYSTER Jr. et al.)<br>* Spalte 1, Zeilen 12-22; Figur 1 *<br>-- | 1 |
| A | DE - A - 2 311 806 (EAST/WEST MEDICAL PRODUCTS)<br>* Seite 2, Zeilen 14-24, Figur 1 *<br>---- | 1 |

**KLASSIFIKATION DER ANMELDUNG (Int.Cl. 3)**

G 01 N 33/12
C 12 Q 1/24

**RECHERCHIERTE SACHGEBIETE (Int. Cl. 3)**

G 01 N 33/12
1/02
C 12 Q 1/24

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 10-12-1979 | ANTHONY |

EPA form 1503.1 06.78